Europäisches Patentamt

European Patent Office

Office européen des brevets

(19)

(11) Publication number: **0 329 268**
**A2**

# EUROPEAN PATENT APPLICATION

(12)

(21) Application number: **89300101.6**

(22) Date of filing: **06.01.89**

(51) Int. Cl.⁴: **A61K 6/08**

The title of the invention has been amended (Guidelines for Examination in the EPO, A-III, 7.3).

(30) Priority: **15.01.88 US 144163**

(43) Date of publication of application:
**23.08.89 Bulletin 89/34**

(84) Designated Contracting States:
**CH DE FR GB IT LI SE**

(71) Applicant: **Kerr Manufacturing Corporation**
**28200 Wick Road Box 455**
**Romulus Michigan 48174(US)**

(72) Inventor: **Jandourek, Emil**
**34202 Burton Lane Livonia**
**Michigan 48154(US)**

(74) Representative: **Oliver, Roy Edward et al**
**POLLAK MERCER & TENCH High Holborn**
**House 52-54 High Holborn**
**London WC1V 6RY(GB)**

(54) **Glass ionomer dental cement curable in two stages.**

(57) A series of dual curing dental cements are disclosed which consist of powder and liquid components to be mixed within a preferred powder to liquid range by weight. Mixing is initiated immediately prior to usage following which the mixed material is applied to the tooth surface as required and cured within a few seconds by irradiation with high intensity visible light energy to a tough leathery consistency, thus permitting the bulk placement of other dental restoratives over it without disturbance. The second stage curing, which is chemically initiated, occurs much more slowly between the powder and the liquid and results in a fully set cement which is hard and rigid.

These cements are adhesive, both to prepared tooth structure and to composite dental restorative materials bulk placed over them. Thus, they combine the advantages of conventional dental glass ionomer cements with those of a first generation visible light cured cavity liner.

EP 0 329 268 A2

# DUAL CURING GLASS IONOMER DENTAL CEMENT

## BACKGROUND OF THE INVENTION

Glass ionomer dental cements were disclosed in British Patent 1,316,129, May 9, 1973. During the ensuing years there have been numerous improvement patents relating to these materials. The first photocured dental cement was disclosed in U.S. Patent 3,629,187, December 21, 1971, and since that date there have been many other improvement patents.

However, to this point in time, there has not been a teaching of a dual curing glass ionomer dental cement which combines the capability for chemical bonding to tooth structure of the glass ionomer dental cement with the capacity for ultrarapid build-up of early physical properties of the photocured dental cement and also chemical bonding to the acrylic functional composite dental filling materials commonly used to complete the filling of a cavity.

## SUMMARY OF THE INVENTION

The dental cement of the present invention is a two component material, and usually, though not necessarily, it exists in the powder plus liquid form wherein the powder contains a basic fluoroalumino silicate glass ionomer component, and the liquid contains water and a polycarboxylate functional moeity with the capability for chemical bonding to tooth structure, though the latter may alternatively be incorporated in the powder, if in finely divided anhydrous form. A photoinitiator is required, and may be incorporated in either the powder or the liquid component, though usually the latter where it can be in solution for rapid maximum reactivity.

Another necessary component is a polymerizable moeity which is capable of being rapidly photocured to a polymeric binder capable of imparting significant early strength properties to the cement while it undergoes a secondary autocuring reaction to attain maximum physical properties and bonding to the tooth structure substrate for interoral performance and longevity.

## DETAILED DESCRIPTION OF THE INVENTION

The material of the present invention consists of two components, preferrably a powder and a liquid, with the following essential composition:

| POWDER | LIQUID |
|---|---|
| 1. Micronized Tonomer Glass<br>2. Radiopaquing Agent<br>3. Photoreducing Agent | 1. Methacrylic Functional Polycarboxylic Acid<br>2. Hydrofunctional Methacrylic Monomer<br>3. Photosensitizer<br>4. Water<br>5. Inhibitor |

The micronized basic fluoroalumino silicate glass is made by melting together a plurality of metallic oxides, plus some metallic fluoride to function as a flux, into a single phase glass of appreciable basicity or alkalinity, due to inclusion of at least one very basic or alkaline metallic oxide. This glass is then fritted and ground to low micron sized particles in a size range of about 0.5 to 10 microns.

When the powder and liquid of the present invention are mixed together they form a paste, which is placed in a tooth cavity other the exposed dentine covering the pulp and irradiated with visible light of high intensity and bout 400-550 nanometers wavelength. This light energy is absorbed by the photosensitizer, which readily under goes scisson in the presence of the photoreducing agent, forming free radicals that initiate copolymerization of the methacrylic functional polycarboxylic acid and the hydroxyfunctional methacrylic monomer and convert the paste to a leathery solid within about 20 seconds.

The secondary setting or curing reaction, which takes up to approximately 24 hours to reach completion, takes place between the carboxylic groups of the methacrylic functional polycarboxylic acid and the micronized basic fluoroalumino silicate glass in the presence of water.

The aqueous polycarboxylic acid attacks the surface of the glass particles, and following a short dissolution stage forms a gel initially, which slowly precipitates a cement that binds the particles together and transforms the leathery mass into a solid inflexible mass.

The essential basic ingredients for the dual curing glass ionomer dental cement of the present invention are:

1) a finely divided basic fluoroalumino silicate glass powder;
2) water;
3) a polyacid, usually polycarboxylate functional;
4) a photoinitiator;
5) a synergistic reducing agent, or free radical accelerator; and
6) a photocurable polymerizable moeity.

Preferably, a radiopaquing agent is included to facilitate radiographic detection of the cement material in vivo, and optionally an accelerator for the glass ionomer/polyacid reaction, plus other desirable additives, such as polymerization inhibitors, to minimize spoilage of the material during storage prior to use, and pigments. It is possible, and has been achieved, to synthesize a series of suitable water soluble polymers, which have both polycarboxylic and readily photopolymerizable functionalities.

These polymers with dual functionality can be synthesized by several different methods, one of which is to first prepare a polymer or a copolymer of an unsaturated carboxylic acid or anhydride with an unsaturated carboxylic acid in any suitable ratio, which is then reacted with an epoxy or hydroxyl functional acrylic functional monomer, in a minor proportion, to form a molecule having both polycarboxylic and readily photopolymerizable acrylic functionality. Suitable unsaturated carboxylic acid anhydrides could be chosen from: Citraconic Anhydride; Maleic Anhydride; Itaconic Anhydride; and Anhydroaconitic Acid.

Unsaturated monocarboxylic acids could be chosen from: Acrylic Acid; Methacrylic Acid; Isocrotonic Acid; Crotonic Acid; Angelic Acid; Cinnamic Acid; Coumaric Acid; Vinylacetic Acid; Isocrotonic Acid; Tiglic Acid; Fumaric Acid; Muconic Acid; Mesaconic Acid; Maleic Acid; Citraconic Acid; Aconitic Acid; and Itaconic Acid.

Epoxy or hydroxyl functional acrylic functional monomers could be chosen from: Glycidyl Acrylate; Glycidyl Methacrylate; Hydroxyethyl Acrylate; Hydroxyproplyl Acrylate; Hydroxyethyl Methacrylate; and Hydroxypropyl Methacrylate.

A further controlled quantity of either one of the latter two low toxicity acrylic functional monomers can also be used as a diluent monomer in the liquid component.

Suitable basic fluoroalumino silicate glass powders which may be used in the present invention are disclosed in U.S. Patents 3,814,717; 4,209,434; 4,360,605; and 4,376,835 which are incorporated herein by reference, or any other suitable permutation of those described of which there are now many commercially available variants. Specific examples of basic fluoroalumino silicate glass powders suitable for specific use with the present invention are as follows:

| Glass Powder No. 1 | |
| --- | --- |
| | WT% |
| Silicon Dioxide | 31 |
| Aluminum Oxide | 24 |
| Soldium Aluminum Fluoride | 18 |
| Aluminum Phosphate | 15 |
| Aluminum Fluoride | 12 |

| Glass Powder No. 2 | |
|---|---|
| | WT% |
| Silicon Dioxide | 30.0 |
| Aluminum Oxide | 16.5 |
| Soldium Aluminum Fluoride | 5.0 |
| Aluminum Phosphate | 10.0 |
| Aluminum Fluoride | 5.3 |
| Calcium Fluoride | 33.2 |

| Glass Powder No. 3 | |
|---|---|
| | WT% |
| Aluminum Oxide | 22 |
| Silicon Dioxide | 38 |
| Aluminum Fluoride | 11 |
| Aluminum Phosphate | 13 |
| Soldium Aluminum Fluoride | 16 |

Two particular basic fluoroalumino silicate glass powders suitable for use in the present invention are available from Industrial Corporation of Glenmore, PA under the designations IG-90-2578 for high reactivity, and IG-90-2589 for lower reactivity.

The water used with formulations of the present invention should be freshly distilled or deionized.

A photoinitiator may be chosen having peak light energy absorption and excitation in the wavelength band between about 400 to 550 nanometers, in order to take maximum advantage of the spectral output of most visible light curing dental units. Suitable photoinitiators include camphroquinone, benzophenone, acetophenone, 4-methoxy acetophenone, diethoxyacetophenone, benzil, and 2,3-pentanedione.

Any soluble synergistic reducing agent or free radical accelerator which is chemically compatible with the liquid or powder component and efficient in conjunction with the chosen photoinitiator may be used, though a soluble salt of an acid which can be dispersed in the powder component and subsequently react with the photoinitiator upon mixing and irradiation to generate free radicals, such as sodium paratoluene sulfinate is a preferred choice. Examples of suitable synergistic reducing agents are: Diethylamino benzaldehyde; Diethylamino butanone; Diethylaminomethyl-2,5-dimethylphenol; Diethylaminophenol; Diethylamino salicylic acid; Diethylaminoethyl methacrylate; Diethylaminoethyl acrylate; Dimethylamino benzaldehyde; Dimethylamino benzoic acid; Dimethylamino benzoin; Dimethylamino benzophenone; Dimethylaminocinnamaldehyde; Dimethylaminoethyl acrylate; Dimethylaminoethyl methacrylate; Dimethylaminophenol; Dimethylaminopropanol; Dimethylaminopropylamine; and Dimethylamino salicylic acid. Those that are solids may be incorporated in the powder component in finely divided form.

Suitable examples of materials for use as radiopaquing agents are: barium aluminum borosilicate; barium sulfate; barium tungstate; strontium aluminum borosilicate; and zinc aluminum borosilicate.

A wide range of chelating agents can be used to accelerate the reaction between the glass powder and the chosen polyacid, or polyacid condensate with ethylenically unsaturated monomers, such as: Citric acid; Dihydroxy tartaric acid; Ethylenediaminetetraacetic acid; Dihydroxybenzoic acid; Pyromellitic acid; Tartaric acid; Tartronic acid; Salicylic acid; and Tetra hydrofuran tetracarboxylicacid. Organometallic chelating agents may also be used, such as aluminum triacetyl acetonate or ethylenediaminetetraacetic acid zinc complex.

The following examples represent various embodiments of the present invention:

EXAMPLE 1

4

| LIQUID COMPONENT | WT% |
|---|---|
| Polyacrylic Acid (MW 5000-15,000) | 40.0 |
| Water | 40.0 |
| Hydroxyethylmethacrylate | 17.5 |
| 4-Methoxyphenol | 0.04 |
| Benzil | 0.3 |
| Citric Acid | 2.16 |
| | 100.00 |

| POWDER COMPONENT | WT% |
|---|---|
| *Glass Powder (10 Micron and below) | 55.0 |
| Barium Sulfate | 44.8 |
| Sodium p-Toluene Sulfinate | 0.2 |
| | 100.00 |

*IG-90-2578 available from Industrial Corporation of Glenmore, PA.

Mixed in a 1.2:1 powder/liquid ratio this formulation gave 1.5 minutes work time. It cured to a Shore A Durometer of 95 in a 1.0 mm layer with 30 seconds exposure to a dental curing unit having an output intensity of greater than 15,000 microwatts per square centimeter at a wavelength of 475-550 nanometers. The hardness increased to Barcol 65 overnight.

EXAMPLE 2

| LIQUID COMPONENT | WT% |
|---|---|
| Acrylic Acid-Maleic Acid Copolymer (equimolar) | 40.0 |
| Water | 40.0 |
| Hydroxypropylmethacrylate | 17.5 |
| 4-Methoxyphenol | 0.04 |
| Benzil | 0.3 |
| Tartaric Acid | 2.16 |
| | 100.00 |

Mixed in a 1.2:1 powder/liquid ratio the powder component of Example 1, this formulation gave 1.75 minutes work time. It cured to a Shore A Durometer of 97 in a 1.0mm layer with 30 seconds exposure to a dental curing unit. Hardness increased to Barcol 68 overnight.

EXAMPLE 2

| LIQUID COMPONENT | WT% |
|---|---|
| Acrylic Acid-Maleic Acid Copolymer Glycidyl Methacrylate Adduct (1:1 by weight) | 45.0 |
| Water | 45.0 |
| Hydroxyethylmethacrylate | 7.5 |
| 4-Methoxyphenol | 0.04 |
| Benzil | 0.3 |
| Tartaric Acid | 2.16 |
| | 100.00 |

Mixed in a 1.2:1 powder/liquid ratio with the powder component of Example 1, this formulation gave 2.0 minutes work time. It cured to Shore A Durometer of 98 in a 1.0mm layer with 30 seconds exposure to a dental curing unit. Hardness increased to Barcol 70 overnight.

## EXAMPLE 4

| POWDER COMPONENT | WT% |
|---|---|
| Glass Powder of Example 1 | 79.8 |
| Barium Tungstate | 20.0 |
| Sodium p-Toluene Sulfinate | 0.2 |
| | 100.00 |

| LIQUID COMPONENT | WT% |
|---|---|
| Acrylic Acid-Maleic Acid Copolymer Glycidyl Methacrylate Adduct (1:1 by weight) | 48.0 |
| Water | 48.0 |
| Hydroxypropylmethacrylate | 1.0 |
| 4-Methoxyphenol | 0.04 |
| 2,3-Bornanedione | 0.25 |
| Citric Acid | 2.71 |
| | 100.00 |

Mixed in a 1.2:1 powder/liquid ratio, this formulation gave 1.75 minutes work time. It cured to Shore A Durometer 95 in a 1.0mm layer with 30 seconds exposure to a dental curing unit. Hardness increased to Barcol 70 overnight.

## EXAMPLE 5

| POWDER COMPONENT | WT% |
|---|---|
| Acrylic Acid-Maleic Acid Copolymer (equimolar) | 45.0 |
| Glass Powder as Example 1 | 44.25 |
| Barium Tungstate | 10.0 |
| Dimethylaminobenzoic acid | 0.4 |
| 2,3-Bornanedione | 0.3 |
| 4-Methoxyphenol | 0.05 |
| | 100.00 |

| LIQUID COMPONENT | WT% |
|---|---|
| Water | 82.5 |
| Tartaric Acid | 2.5 |
| Hydroxypropylmethacrylate | 5.0 |
| | 100.00 |

Mixed in a 1.2:1 powder/liquid ratio, this formulation gave 2.25 minutes work time. It cured to a Shore A Durometer 94 in a 1.0mm layer with 30 seconds exposure to a dental curing unit. Hardness increased to Barcol 65 overnight.

## EXAMPLE 6

| POWDER COMPONENT | WT% |
|---|---|
| Polyacrylic Acid (MW 5000-15,000) | 45.0 |
| Glass Powder of Example 1 | 44.25 |
| Barium Tungstate | 10.0 |
| Dimethylamino Benzaldehyde | 0.4 |
| Benzil | 0.3 |
| 4-Methoxyphenol | 0.05 |

Mixed in a 1.2:1 powder/liquid ratio with the liquid component of Example 5, this formulation gave 2.25 minutes work time. It cured to a Shore A Durometer 95 in a 1.0mm layer with 30 seconds exposure to a dental curing unit. Hardness increased to Barcol 67 overnight.

## EXAMPLE 7

### Method of Preparation of the Powder and Liquid Components

#### Powder

Using the powder components of Example 4, the sodium para toluene sulfinate is first dispersed in the barium tungstate by ballmilling for two hours. This dispersion is then put into a twin shell V-blender together with the basic fluoroalumino silicate glass powder and blended together for one hour. After blending the completed powder it is screened through 325 mesh nylon cloth to break up or remove any agglomerates prior to packaging.

#### Liquid

Using the liquid components of Example 3, the hydroxyethyl methacrylate is placed in a suitable container, such as a large beaker or flask, fitted with an efficient stirrer, and the 4-methoxyphenol and benzil dissolved in it. The tartaric acid and acrylic-maleic acid-glycidyl methacrylate copolymer are dissolved in the water with stirring and gentle heat to approximately 40° C. Finally the hydroxyethyl methacrylate blend is added and homogenized by stirring prior to filling in bottles.

When required for use, the powder:liquid components are spatulated together to form a paste in a preferred weight ratio of about 1.2:1. Mixing is initiated immediately prior to usage following which the mixed material is applied to the tooth surface as required and cured within a few seconds by irradiation with high intensity visible light energy to a tough leathery consistency, thus permitting the bulk placement of other dental restoratives over it without disturbance. The second stage curing, which is chemically initiated, occurs much more slowly between the powder and the liquid and results in a fully set cement which is hard and rigid. These cements are adhesive, both to prepared tooth structure and to composite dental restorative materials bulk placed over them. Thus, they combine the advantages of conventional dental glass ionomer cements with those of a first generation visible light cured cavity liner.

The following illustrates suitable compositional ranges for the powder and liquid components of the present invention:

| HYDRATED POLYACID LIQUID COMPONENT | WT% |
| --- | --- |
| Unsaturated Polyacid | 30-60 |
| Water | 30-60 |
| Hydroxy Functional Acrylic Functional Monomer | 0-15 |
| Inhibitor | 0.005-0.5 |
| Photoinitiator | 0.01-1.0 |
| Setting Accelerator | 0.05-5.0 |

| POWDER COMPONENT (FOR USE WITH ABOVE LIQUIDS) | WT% |
| --- | --- |
| Basic Fluoroalumino Silicate Glass Powder | 40-80 |
| Radiopaquing Agent | 15-55 |
| Synergistic Reducing Agent For Free Radical Generator | 01.-1.0 |

| ANHYDROUS POLYACID POWDER COMPONENT | WT% |
| --- | --- |
| Anhydrous Polyacid | 30-60 |
| Basic Fluoroalumino Silicate Glass Powder | 30-60 |
| Radiopaquing Agent | 15-40 |
| Solid Synergistic Reducing Agent For Free Radical Generator | 0.1-1.0 |
| Photoinitiator | 0.01-1.0 |
| Inhibitor | 0.005-0.5 |

| LIQUID COMPONENT (FOR USE WITH ABOVE POWDERS) | WT% |
| --- | --- |
| Water | 80-95 |
| Setting Accelerator | 1-10 |
| Acrylic Functional Hydroxy Functional Monomer | 1-15 |

The following illustrates suitable compositional ranges for a cured cement of the present invention:

|  | WT% |
|---|---|
| Basic Fluoroalumino Silicate Glass Powder | 20-60 |
| Saturated or Unsaturated Polycarboxylic Acid | 20-60 |
| Water | 20-60 |
| Radiopaquing Agent | 5-50 |
| Synergistic Reducing Agent or Free Radical Generator | 0.05-1.0 |
| Photoinitiator | 0.005-1.0 |
| Inhibitor | 0.005-0.25 |
| Setting Accelerator | 0.5-5.0 |
| Hydroxy Functional Acrylic Functional Monomer | 0.01-5.0 |

Although particular embodiments of the present invention have been disclosed herein for purposes of explanation, further modifications or variations thereof will be apparent to those skilled in the art to which this invention pertains.

## Claims

1. A two component dual curing dental cement which comprises:

| Powder Component | Liquid Component |
|---|---|
| Basic Fluoroalumino Silicate Glass Powder<br>Radiopaquing Agent<br>Photoreducing Agent | Methyacrylic Functional Polycarboxylic Acid<br>Hydroxyfunctional Methacrylic Monomer<br>Photosensitizer<br>Inhibitor<br>Water |

2. A two component dual curing dental cement which comprises:

| Powder Component | Wt% | Liquid Component | Wt% |
|---|---|---|---|
| Basic Fluoroalumino Silicate Glass Powder | 20-60 | Methacrylic Functional Polycarboxylic Acid | 20-60 |
| Radiopaquing Agent | 15-50 | Hydrofunctional Methacrylic Monomer | 0.01-5.0 |
| Photoreducing Agent | 0.1-1.0 | α Diketone Photosensitizer | 0.05-1.0 |
|  |  | Inhibitor | 0.005-0.25 |
|  |  | Deionized Water | 20-60 |

3. A two component dual curing dental cement which comprises:

| Liquid Component | Wt% |
|---|---|
| Polyacrylic Acid (MW 5000-15,000) | 40.0 |
| Water | 40.0 |
| Hydroxyethylmethacrylate | 17.5 |
| 4-Methoxyphenol | 0.04 |
| Benzil | 0.3 |
| Citric Acid | 2.16 |

| Powder Component | Wt% |
|---|---|
| Basic Fluoroalumino Silicate Glass Powder (10 Micron and below) | 55.0 |
| Barium Sulfate | 44.8 |
| Sodium p-Toluene Sulfinate | 0.2 |

4. A dual curing two component dental cement which comprises:

| Hydrated Polyacid Liquid Component | WT% |
|---|---|
| Unsaturated Polyacid | 30-60 |
| Water | 30-60 |
| Hydroxy Functional Acrylic Functional Monomer | 0-15 |
| Inhibitor | 0.005-0.5 |
| Photoinitiator | 0.01-1.0 |
| Accelerator | 0.05-5.0 |

| Powder Component | WT% |
|---|---|
| Basic Fluoroalumino Silicate Glass Powder | 40-80 |
| Radiopaquing Agent | 15-55 |
| Reducing Agent For Free Radical Generator | 0.1-1.0 |

5. A dual curing two component dental cement which comprises:

| Anhydrous Polyacid Powder Component | WT% |
|---|---|
| Anhydrous Polyacid | 30-60 |
| Basic Fluoroalumino Silicate Glass Powder | 30-60 |
| Radiopaquing Agent | 15-40 |
| Solid Synergistic Reducing Agent or Free Generator | 0.1-1.0 |
| Photoinitiator | 0.01-1.0 |
| Inhibitor | 0.005-0.5 |

| Liquid Component | WT% |
|---|---|
| Water | 80-95 |
| Accelerator | 1-10 |
| Acrylic Functional Hydroxy Functional Monomer | 1.15 |

6. A cured dental cement which comprises a finely divided basic fluoroalumino silicate glass powder, water, a polycarboxylate functional polyacid, a photoinitiator, a free radical generator, and a photocurable polymerizable moeity.

7. A cured glass ionomer dental cement which comprises:

|  | Wt% |
|---|---|
| Basic Fluoroalumino Silicate Glass Powder | 20-60 |
| Unsaturated Polycarboxylic Acid | 20-60 |
| Water | 20-60 |
| Radiopaquing Agent | 5-50 |
| Reducing Agent or Free Radical Generator | 0.05-1.0 |
| Photoinitiator | 0.005-1.0 |
| Inhibitor | 0.005-0.25 |
| Accelerator | 0.5-5.0 |
| Hydroxy Functional Acrylic Functional Monomer | 0-5.0 |

8. A cured dual curing dental cement which comprises:

|  | WT% |
|---|---|
| Basic Fluoroalumino Silicate Glass | 43 |
| Unsaturated Polycarboxylic Acid | 24 |
| Water | 24 |
| Radiopaquing Agent | 7.25 |
| Reducing Agent or Free Radical Generator | 0.1 |
| Photoinitiator | 0.01 |
| Inhibitor | 0.1 |
| Accelerator | 1.5 |
| Hydroxy Functional Acrylic Functional Monomer | 0.04 |
|  | 100.00 |